**Europäisches Patentamt**

**European Patent Office**

**Office européen des brevets**

(11) Veröffentlichungsnummer: **0 236 864**

**A1**

## (12) EUROPÄISCHE PATENTANMELDUNG

(21) Anmeldenummer: **87102738.9**

(51) Int. Cl.4: **A61B 6/00**

(22) Anmeldetag: **26.02.87**

(30) Priorität: **11.03.86 DE 3608041**

(43) Veröffentlichungstag der Anmeldung:
**16.09.87 Patentblatt 87/38**

(84) Benannte Vertragsstaaten:
**DE FR**

(71) Anmelder: **Siemens Aktiengesellschaft Berlin und München**
**Wittelsbacherplatz 2**
**D-8000 München 2(DE)**

(72) Erfinder: **Jürgens, Hans, Dipl.-Ing.(FH)**
**Sieglitzhoferstrasse 44**
**D-8520 Erlangen(DE)**

(54) **Fahrbares Röntgenuntersuchungsgerät.**

(57) Die Erfindung betrifft ein fahrbares Röntgenuntersuchungsgerät mit einem C-Bogen (1), der an seinen Enden einen Röntgenstrahler (2) und einen Strahlenempfänger (3) trägt und auf einem Wagen (7) verstellbar gelagert ist, welcher einen horizontal liegenden Ausleger (6) aufweist, wobei der C-Bogen (1) in einem Halter (4) längs seines Umfanges verstellbar gelagert ist. Der Halter (4) ist auf dem Ausleger (6) vorzugsweise über ein Scherengestänge (5) höhenverstellbar angeordnet. Die Kabel für Röntgenstrahler (2) und Strahlenempfänger (3) sind unsichtbar im C-Bogen (1) geführt.

FIG 1

EP 0 236 864 A1

## Fahrbares Röntgenuntersuchungsgerät

Die Erfindung betrifft ein fahrbares Röntgenuntersuchungsgerät mit einem C-Bogen, der an seinen Enden einen Röntgenstrahler und einen Strahlenempfänger trägt und auf einem Wagen verstellbar gelagert ist, welcher einen- horizontal liegenden Ausleger aufweist, wobei der C-Bogen in einem Halter längs seines Umfanges verstellbar geführt ist.

Röntgenuntersuchungsgeräte dieser Art erlauben eine universelle Einstellung des Röntgenstrahlenganges im Raum und werden bei der Durchführung von Operationen zur Röntgenkontrolle angewandt. Der C-Bogen umgreift dabei den zu durchstrahlenden Körperteil.

Der Erfindung liegt die Aufgabe zugrunde, ein Röntgenuntersuchungsgerät der eingangs genannten Art so auszubilden, daß durch eine einfache Mechanik sowohl eine Höhenverstellung als auch eine Verstellung des C-Bogens längs seines Umfanges ermöglicht ist.

Diese Aufgabe ist erfindungsgemäß dadurch gelöst, daß der Halter für den C-Bogen auf dem Ausleger höhenverstellbar angeordnet ist. Durch einen einfachen Höhenverstellmechanismus zwischen dem Halter und dem Ausleger des Wagens ist dabei eine Höhenverstellung des C-Bogens mit dem Röntgenstrahler und dem Strahlenempfänger möglich. Der Höhenverstellmechanismus kann in einfacher Weise von einem Scherengestänge gebildet sein.

Eine besonders zweckmäßige Ausgestaltung ergibt sich, wenn die Kabel für Röntgenstrahler und Strahlenempfänger im C-Bogen geführt sind, wobei im Halter eine Rolle zum gegenläufigen Auf-und Abwickeln der Kabel angeordnet ist. Bei dieser Ausführung ist auf frei zum Röntgenstrahler und zum Strahlenempfänger verlegte Kabel verzichtet.

Die Erfindung ist nachfolgend anhand eines in der Zeichnung dargestellten Ausführungsbeispieles näher erläutert. Es zeigen:

Fig. 1 ein Röntgenuntersuchungsgerät nach der Erfindung, und

Fig. 2 und 3 die Kabelführung des Röntgenuntersuchungsgerätes gemäß Figur 1.

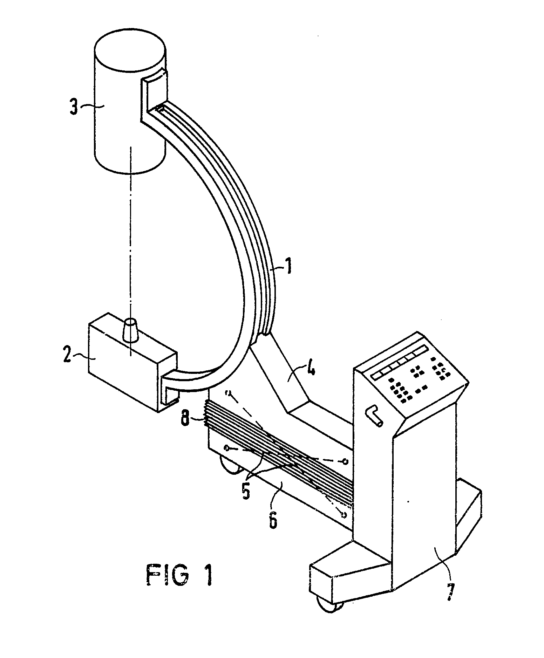

In der Figur 1 ist ein C-Bogen 1 dargestellt, der an seinen Enden einen Röntgenstrahler 2 und einen Röntgenbildverstärker 3 trägt. Der Patient liegt bei einer Röntgenuntersuchung zwischen dem Röntgenstrahler 2 und dem Röntgenbildverstärker 3, d.h. der C-Bogen 1 umgreift dabei den zu untersuchenden Körperteil des Patienten. Der C-Bogen 1 ist an einem Halter 4 längs seines Umfanges verstellbar gelagert, welcher über ein Scherengestänge 5 mit einem Ausleger 6 eines Wagens 7 verbunden ist. Der Wagen 7 trägt drei Räder zum Verfahren des Röntgenuntersuchungsgerätes auf dem Fußboden. Eines dieser Räder ist am Ausleger 6 angeordnet.

Der C-Bogen 1 ist mit dem Röntgenstrahler 2 und dem Röntgenbildverstärker 3 zur Einstellung des Röntgenstrahlenganges einerseits längs seines Umfanges gegenüber dem Halter 4 verstellbar und andererseits zusammen mit dem Halter 4 gegenüber dem Ausleger 6 und damit dem Wagen 7 höhenverstellbar. Das Scherengestänge 5 ist dabei von einer Faltenhülle 8 umgeben.

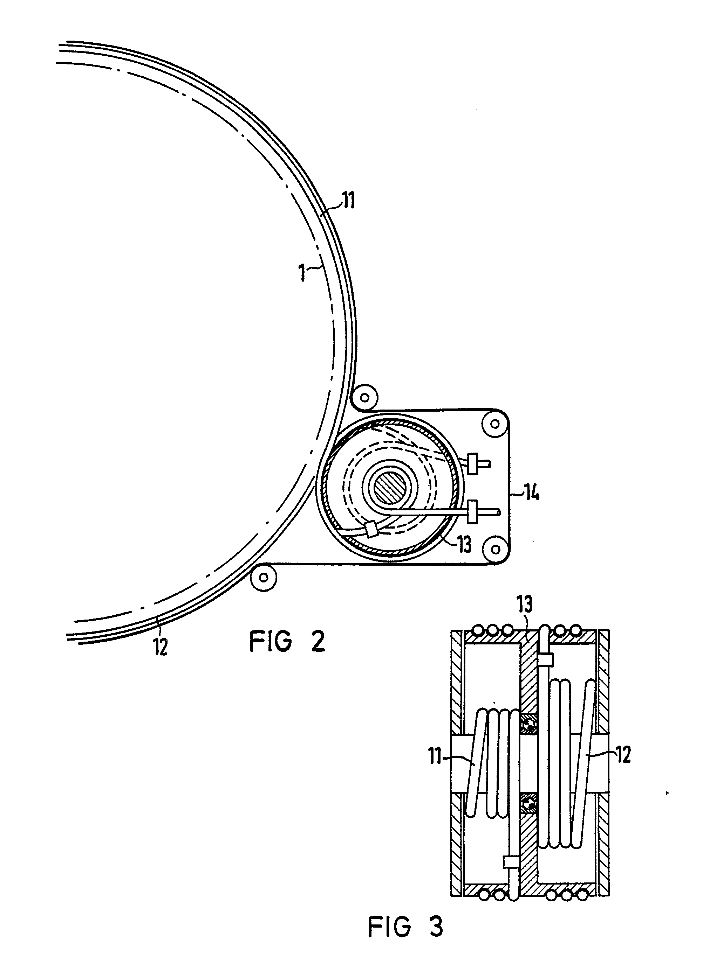

Die Kabel zum Röntgenstrahler 2 und zum Röntgenbildver stärker 3 sind im Innern des C-Bogens 1 unsichtbar geführt. Die Figuren 2 und 3 zeigen die wesentlichen Elemente der Kabelführung. In der Figur 2 ist der C-Bogen 1 - schematisch dargestellt. Das Kabel 11 führt zum Röntgenbildverstärker 3 und das Kabel 12 zum Röntgenstrahler 2. Die Kabel 11, 12 sind gegenläufig auf einer in der Figur 2 nur schematisch dargestellten Kabeltrommel 13 aufgewickelt. Die Figur 3 zeigt die Kabeltrommel 13 genauer. Die Kabeltrommel 13 liegt im Halter 4. Aufgrund der gegenläufigen Kabelwicklung rollt sich beim Verstellen des C-Bogens 1 gegenüber dem Halter 4 eines der Kabel 11, 12 von der Kabeltrommel 13 ab und das andere wird aufgerollt. Es treten also keine Kabelschlaufen im Innern des Gerätes auf. Die Figur 2 zeigt, daß die Kabeltrommel 13 nach außen durch ein über Rollen geführtes Abdeckband 14 abgedeckt ist, das am C-Bogen 1 befestigt ist.

## Ansprüche

1. Fahrbares Röntgenuntersuchungsgerät mit einem C-Bogen (1), der an seinen Enden einen Röntgenstrahler (2) und einen Strahlenempfänger - (3) trägt und auf einem Wagen (7) verstellbar gelagert ist, welcher einen horizontal liegenden Ausleger (6) aufweist, wobei der C-Bogen (1) in einem Halter (4) längs seines Umfanges verstellbar geführt ist, **dadurch gekennzeichnet**, daß der Halter (4) auf dem Ausleger (6) höhenverstellbar angeordnet ist.

2. Röntgenuntersuchungsgerät nach Anspruch 1, **dadurch gekennzeichnet**, daß der Höhenverstellmechanismus von einem Scherengestänge (5) gebildet ist.

3. Röntgenuntersuchungsgerät nach Anspruch 1 oder 2, **dadurch gekennzeichnet**, daß die Kabel (11, 12) für Röntgenstrahler (2) und Strahlenempfänger (3) im C-Bogen (1) und im Halter (4) geführt sind, wobei im Halter (4) eine Rolle (13) zum gegenläufigen Auf-und Abwickeln der Kabel (11, 12) angeordnet ist.

FIG 1

FIG 2

FIG 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-1 175 032 (ETABLISSEMENTS DE MAN) * Figuren 1-3; Seite 1, Zeile 75 - Seite 2, Zeile 93 * | 1 | A 61 B 6/00 |
| A | --- | 3 | |
| A | NL-C- 59 144 (ATSE GERRITS HOEKSTRA) * Insgesamt * --- | 2 | |
| A | US-A-4 065 978 (MERESZ) * Figuren; Spalte 3, Zeile 15 - Spalte 5, Zeile 55 * ----- | 3 | |

RECHERCHIERTE SACHGEBIETE (Int. Cl.4)

A 61 B

Der vorliegende Recherchenbericht wurde für alle Patentansprüche erstellt.

| Recherchenort DEN HAAG | Abschlußdatum der Recherche 15-06-1987 | Prüfer CHEN A.H. |
|---|---|---|